# EUROPÄISCHE PATENTSCHRIFT

(11) **EP 4 382 886 B1**
(45) Veröffentlichungstag und Bekanntmachung des Hinweises auf die Patenterteilung: **11.11.2026**
(21) Anmeldenummer: 22211799.6
(22) Anmeldetag: 06.12.2022
(51) Int. Cl.: G01N 1/22

(54) **SYSTEM UND ANORDNUNG ZUR VERMESSUNG EINES IN EINER GASLEITUNG STRÖMENDEN GASES**
SYSTEM AND ARRANGEMENT FOR MEASURING A GAS FLOWING IN A GAS LINE
SYSTÈME ET DISPOSITIF DE MESURE D'UN GAZ S'ÉCOULANT DANS UNE CONDUITE DE GAZ

(43) Veröffentlichungstag der Anmeldung: 12.06.2024
(73) Patentinhaber: Meter-Q Solutions GmbH, 61169 Friedberg (DE)
(72) Erfinder: Suhr, Jan, 35510 Butzbach (DE); Zajc, Achim, 35510 Butzbach (DE)
(74) Vertreter: Heeschen Pültz Patentanwälte PartGmbB

(56) Entgegenhaltungen:
- EP-A1- 0 523 396
- US-A- 3 938 390
- US-A1- 2002 048 818
- US-B2- 8 084 263

## Beschreibung

Die Erfindung betrifft ein System und eine Anordnung zur Beschaffenheitsmessung eines in einer Gasleitung strömenden Gases.

Bei Gasleitungen, insbesondere Gasleitungen von Erdgasnetzen ist es notwendig, an einem oder mehreren Messpunkten eine Beschaffenheitsmessung des in der Gasleitung strömenden Gases durchzuführen. Dabei strömt das Gas in solchen Gasleitungen mit hohen Geschwindigkeiten und einem hohen Druck, um einen schnellen Transport des Gases über das entsprechende Netz zu ermöglichen. Typische Strömungsgeschwindigkeiten können zwischen 2 bis 35 m/s und ein typischer Druck kann zwischen 5 bis 100 barg betragen.

Zur Beschaffenheitsmessung können entsprechende Messgeräte verwendet werden. Diese weisen in der Regel eine Entnahmesonde auf, welche in der Gasleitung anordenbar ist und Gas entnimmt. Bei einem hohen Druck des Gases in der Leitung weist das Gas in der Entnahmesonde ebenfalls einen hohen Druck auf und muss je nach Messvorrichtung zunächst über eine Druckreduziervorrichtung geleitet werden, um den Druck zu reduzieren. Im Anschluss wird das Gas zu der Messvorrichtung beispielsweise zum Messen der Beschaffenheit geleitet. Typischerweise wird nur ein Anteil des Gases zur Beschaffenheitsmessung verwendet. Der restliche Anteil sowie das vermessene Gas werden an die Umgebung abgegeben. Eine Rückführung in die Gasleitung ist nicht vorgesehen, da diese mit bisherigen bekannten Mitteln nicht wirtschaftlich ist. Das aus der Gasleitung entnommene Gas müsste zunächst gesammelt und anschließend der Druck des gesammelten Gases erhöht werden, um das Gas zurück in die Gasleitung führen zu können. Dies würde bedeuten, dass weitere Einheiten, mehr Bauraum und Energie benötigt werden würden. Entsprechend kommt es zum einen zu einem Verlust an Gas in der Leitung und zum anderen zu einer erhöhten Umweltbelastung durch den Ausstoß des Gases.

Stand der Technik ist aus US 2002/048818 A1, EP 0 523 396 A1 und US 8 084 263 B2 bekannt.

Es ist eine Aufgabe der vorliegenden Erfindung, einen oder mehrere der zuvor genannten Nachteile zu beheben und/oder zumindest zu verbessern. Es ist insbesondere eine Aufgabe der vorliegenden Erfindung, zumindest einen Anteil des entnommenen Gases zurückzuführen und die Umweltbelastung zu verringern, indem der Anteil des an die Umwelt abzugebenden Gases reduziert wird.

Die Aufgabe wird gemäß einem ersten Aspekt durch ein System zur Vermessung eines in einer Gasleitung strömenden Gases gelöst, umfassend eine Entnahmesonde mit einer Eingangsöffnung zum Entnehmen eines in einer Gasleitung strömenden Gases, einer Ausgangsöffnung zum zumindest teilweisen Ausgeben des entnommenen Gases in die Gasleitung und einer Entnahmeleitung, die zumindest die Eingangsöffnung und die Ausgangsöffnung fluidisch verbindet. Die Entnahmesonde ist derart ausgebildet, dass zumindest die Eingangsöffnung und die Ausgangsöffnung in der Gasleitung anordenbar sind, wobei die Entnahmesonde derart ausgebildet ist, dass die Ausgangsöffnung entlang einer Strömungsrichtung des in der Gasleitung strömenden Gases stromabwärts der Eingangsöffnung anordenbar ist. Das System umfasst weiter zumindest eine Messvorrichtung, wobei die Entnahmeleitung eine Schnittstelle aufweist, über die zumindest teilweise das Gas zu der Messvorrichtung zum Vermessen des Gases leitbar ist.

Das Gas in der Gasleitung kann mit einem hohen Druck und/oder einer hohen Strömungsgeschwindigkeit bereitgestellt sein. Der Druck des Gases in der Gasleitung kann zwischen 5 bis 100 barg betragen. Die Strömungsgeschwindigkeit des Gases in der Gasleitung kann zwischen 2 bis 35 m/s betragen. Mittels der Entnahmesonde kann eine "Loop" bzw. ein Kreislauf bereitgestellt werden, bei dem das Gas insbesondere mit dem hohen Druck und/oder der hohen Strömungsgeschwindigkeit von der Eingangsöffnung von der Gasleitung entnommen wird und das Gas über die Entnahmeleitung zu der Ausgangsöffnung geleitet werden kann. Mittels der Schnittstelle kann ein Anteil des entnommenen Gases aus der Entnahmeleitung entnommen und zu der Messvorrichtung zur Vermessung des Gases geleitet werden. Entsprechend kann bereits bei der Schnittstelle nur ein reduzierter Anteil des Gases entnommen und zu der Messvorrichtung oder einer nachfolgend definierten Druckreduziervorrichtung zur Reduzierung eines Drucks des entnommenen Gases geleitet entnommen und zu der Messvorrichtung oder einer nachfolgend definierten Druckreduziervorrichtung zur Reduzierung eines Drucks des entnommenen Gases geleitet werden. Der restliche Anteil des Gases in der Entnahmeleitung mit hohem Druck kann zurück in die Gasleitung geleitet werden. Entsprechend wird die Menge an Gas, welche zum Vermessen entnommen wird, reduziert. Weiter ist an der Lösung vorteilhaft, dass der Druck und/oder die Strömungsgeschwindigkeit des Gases in der Gasleitung genutzt werden. Durch die Anordnung der Eingangs- und Ausgangsöffnungen in der Gasleitung kann eine Druckdifferenz zwischen der Eingangs- und Ausgangsöffnung entstehen, insbesondere durch einen Staudruck an der Eingangsöffnung und eine dynamische Druckreduzierung an der Ausgangsöffnung. Diese Druckdifferenz kann dazu führen, dass das Gas über die Eingangsöffnung entnommen, über die Entnahmeleitung und zu der Ausgangsöffnung strömt, da die Druckdifferenz zu einer Strömung bzw. einer Strömungsgeschwindigkeit innerhalb der Entnahmesonde führen kann. Beispielsweise kann der Druck des Gases innerhalb der Gasleitung 100 bar aufweisen, sodass es zu einem Staudruck von 5 bar des Gases an der Eingangsöffnung, somit zu einem Gesamtdruck von 105 bar an der Eingangsöffnung und zu einem dynamischen Druck von 2 bar des Gases an der Ausgangsöffnung, somit zu einem Gesamtdruck von 98 bar an der Ausgangsöffnung kommen kann. Somit liegt eine Druckdifferenz von 7 bar zwischen der Eingangs- und Ausgangsöffnung vor.

Basierend auf dem Druck des Gases in der Gasleitung, einer nachfolgend definierten Strömungsstrecke und/oder einer Messaufgabe der Messvorrichtung kann auf vorteilhafte Weise Gas aus der Gasleitung mittels der Entnahmesonde entnommen, teilweise zu der Messvorrichtung geleitet und teilweise zu der Ausgangsöffnung geleitet werden, wobei nur eine für eine Vermessung der Messvorrichtung notwendige Gasmenge mittels der Schnittstelle aus der Entnahmeleitung entnommen wird und der restliche Anteil zurück in die Gasleitung ausgegeben werden kann. Weiter kann das entnommene Gas basierend auf dem Druck das Gases in der Gasleitung auf natürliche Art und Weise, somit ohne weitere Mittel, entlang einer vorbestimmten Strömungsstrecke mit einer vorbestimmten Strömungsgeschwindigkeit strömen. Entsprechend kann zumindest die Messvorrichtung beabstandet zu der Gasleitung angeordnet werden. Eine Beabstandung der Messvorrichtung kann in vorteilhafterweise weiter durch das Bereitstellen einer Pumpe erhöht werden, welche dazu ausgebildet ist, zumindest teilweise eine vorbestimmte Druckdifferenz in der Entnahmesonde bereitzustellen. Entsprechend kann eine Distanz zwischen der Gasleitung und der Vorrichtung, insbesondere der Druckreduziervorrichtung vergrößert werden, ohne Einbußen in der Mess- bzw. Strömungsgeschwindigkeit des entnommenen Gases und damit der Vermessungszeit hinnehmen zu müssen.

Stromaufwärts und/oder Stromabwärts kann eine Position einer Einheit und/oder eines Bestandteils des Systems entlang eines Stroms des Gases umfassen und/oder kennzeichnen. Das Gas strömt entlang der Strömungsrichtung in der Gasleitung. Weiter kann das Gas entlang einer Leitung, beispielsweise der Entnahmeleitung von der Eingangsöffnung zu der Ausgangsöffnung, wobei die Richtung des in der Entnahmeleitung strömenden Gases unterschiedlich zu der Strömungsrichtung sein kann. Die Entnahmesonde kann derart ausgebildet sein, dass die Ausgangsöffnung entlang der Strömungsrichtung des Gases, insbesondere des in der Gasleitung strömenden Gases stromabwärts, insbesondere stromabwärts der Eingangsöffnung, und die Eingangsöffnung stromaufwärts, insbesondere stromaufwärts der Ausgangsöffnung, in Bezug auf die Strömung und/oder Strömungsrichtung des Gases in der Gasleitung anordenbar sind.

Die Entnahmesonde, insbesondere die Eingangsöffnung kann derart ausgebildet und in der Gasleitung anordenbar sein, dass das in der Gasleitung strömende Gas aufgrund der Strömungsgeschwindigkeit des Gases mittels der Eingangsöffnung aus der Gasleitung entnehmbar ist.

Die Entnahmesonde ist derart ausgebildet, dass die Druckdifferenz zwischen einem ersten Druck des mittels der Entnahmesonde entnommenen Gases an der Eingangsöffnung und einem zweiten Druck des mittels der Entnahmeleitung geleiteten Gases an der Ausgangsöffnung ausbildbar ist. Die Druckdifferenz ist derart ausbildbar, dass das mittels der Entnahmesonde entnommene Gas mit einer vorbestimmten Strömungsgeschwindigkeit durch die Entnahmesonde strömt. Der erste Druck ist dabei ein Staudruck und der zweite Druck ein dynamischer Druck.

Der erste Druck kann ein Druck sein, den das Gas in einem Entnahmequerschnitt aufweist, der die Eingangsöffnung ausbildet. Der zweite Druck kann ein Druck sein, den das Gas in einem Ausgabequerschnitt aufweist, der die Ausgangsöffnung ausbildet. Der jeweilige Druck kann ein Druck sein, der an einem Messpunkt gemessen wird, beispielsweise an der Eingangsöffnung.

Die Eingangsöffnung kann einen ersten Endabschnitt der Entnahmesonde und/oder die Ausgangsöffnung kann einen zweiten Endabschnitt der Entnahmesonde ausbilden.

Die vorbestimmte Strömungsgeschwindigkeit kann basierend auf der Strömungsstrecke und/oder der Messaufgabe der Messvorrichtung bestimmt sein. Die Messaufgabe der Messvorrichtung kann eine zeitliche Messaufgabe sein, bei der innerhalb eines vorbestimmten Zeitintervalls jeweils eine Vermessung des Gases erfolgt. Dieses Zeitintervall kann 10 Sekunden betragen, sodass eine Vermessung alle 10 Sekunden erfolgt. Alternativ oder zusätzlich kann eine Einmal-Messung erforderlich sein, die innerhalb einer vorbestimmten Zeitdauer erfolgt. Dies kann eine schnelle Messung sein. Entsprechend kann das System, insbesondere die Entnahmesonde derart ausgebildet sein, dass das Gas aus der Gasleitung entnommen und innerhalb von 10 Sekunden, aufgrund einer entsprechenden Strömungsgeschwindigkeit des Gases entlang der Entnahmesonde und möglicher weiterer Leitungen und Einheiten des Systems zu der Messvorrichtung strömt. Sollte durch die Entnahmesonde und den Druck das Gases in der Gasleitung es nicht möglich sein, dass das Gas innerhalb der 10 Sekunden zu der Messvorrichtung strömt, kann eine Pumpe vorgesehen sein, welche die Druckdifferenz bereitstellt und/oder erhöht, sodass die Strömungsgeschwindigkeit ausbildbar ist, derart, dass das Gas innerhalb der 10 Sekunden zu der Messvorrichtung strömt.

Die Strömungsstrecke kann zumindest eine Kreislaufstrecke und/oder eine Messstrecke umfassen, wobei die Kreislaufstrecke eine Strecke ist, die das mittels der Eingangsöffnung entnommene Gas von der Eingangsöffnung entlang der Entnahmeleitung zu der Ausgangsöffnung strömt, wobei die Messstrecke eine Strecke ist, die das mittels der Eingangsöffnung entnommene Gas von der Eingangsöffnung zu der der Messvorrichtung strömt.

Die Eingangsöffnung kann durch den Entnahmequerschnitt zum Entnehmen des Gases aus der Gasleitung und/oder die Ausgangsöffnung durch den Ausgabequerschnitt zum zumindest teilweisen Ausgeben des entnommenen Gases in die Gasleitung ausgebildet sein, wobei die Entnahmesonde derart ausgebildet sein kann, dass der Entnahmequerschnitt und/oder der Ausgabequerschnitt senkrecht zu der Strömungsrichtung in der Gasleitung anordenbar sind. Der Entnahmequerschnitt und/oder der Ausgabequerschnitt können sich folglich jeweils innerhalb einer Ebene erstrecken, die senkrecht zu der Strömungsrichtung angeordnet ist. Alternativ kann sich die Eingangsöffnung und/oder die Ausgangsöffnung, insbesondere der Entnahmequerschnitt und/oder der Ausgabeabschnitt jeweils in einer Ebene erstrecken, die sich nicht senkrecht zu der Strömungsrichtung erstreckt und/oder relativ zu der Strömungsrichtung verkippt ist. Insbesondere können der Entnahmequerschnitt und/oder der Ausgabequerschnitt derart ausgebildet und in der Gasleitung anordenbar sein, dass diese parallel zueinander ausgebildet sind.

Die Entnahmesonde kann derart ausgebildet sein, dass der Entnahmequerschnitt dem entlang der Strömungsrichtung in der Gasleitung strömenden Gas zugewandt, insbesondere derart, dass das in der Gasleitung strömende Gas aufgrund der Strömungsrichtung in die Eingangsöffnung strömt, und/oder der Ausgabequerschnitt dem entlang der Strömungsrichtung in der Gasleitung strömenden Gas abgewandt ist, insbesondere derart, dass das in der Entnahmeleitung strömende Gas aus der Ausgangsöffnung in Richtung der Strömungsrichtung strömt und/oder ausgebbar ist. Die Ausgangsöffnung, insbesondere der Ausgabeabschnitt kann derart anordenbar sein, dass diese bzw. dieser in Richtung der Strömungsrichtung orientiert und/oder geöffnet ist, während die Eingangsöffnung, insbesondere der Entnahmequerschnitt derart anordenbar sein kann, dass diese bzw. dieser entgegen der Strömungsrichtung orientiert und/oder geöffnet ist. Durch diesen Aufbau wird es ermöglicht, dass das in der Gasleitung strömende Gas aufgrund seiner Strömungsgeschwindigkeit frontal auf die Eingangsöffnung trifft, in die Eingangsöffnung strömt und durch die Entnahmeleitung weiter strömt. Dabei kann das Gas durch die Entnahmeleitung eine Richtungsänderung erfahren, die zumindest abschnittsweise unterschiedlich zu der Strömungsrichtung ist. Das Gas strömt über die Entnahmeleitung zumindest teilweise zu der Ausgabeöffnung und kann über die Ausgabeöffnung ausgegeben werden. Dabei kann die Richtung des in der Entnahmeleitung strömenden Gases bei der Ausgabeöffnung und/oder einem Endabschnitt der Entnahmeleitung, welcher die Ausgabeöffnung umfasst, derart abänderbar sein, dass die Richtung des in der Entnahmeleitung strömenden Gases in Richtung der Strömungsrichtung abänderbar ist, insbesondere parallel zu der Strömungsrichtung ist. Das in der Entnahmeleitung strömende Gas kann mittels der Ausgabeöffnung in Richtung der Strömungsrichtung ausgegeben werden, insbesondere wenn die Ausgabeöffnung der Strömungsrichtung abgewandt ist und somit in Richtung der Strömungsrichtung geöffnet ist. In Kombination mit der senkrecht zu der Strömungsrichtung angeordneten Anordnung der Entnahme- und/oder Ausgabequerschnitte können insbesondere der Entnahmen- und/oder der Ausgabequerschnitt um 180° entgegengesetzt zu der Strömungsrichtung angeordnet sein.

Die Eingangs- und/oder Ausgangsöffnung, insbesondere die Entnahme- und/oder Ausgabequerschnitte können rund, insbesondere kreisrund ausgebildet sein.

Die Entnahmesonde kann zumindest abschnittsweise bogenförmig und/oder U-förmig ausgebildet sein, wobei die Eingangs- und/oder Ausgangsöffnungen insbesondere einen jeweiligen Endabschnitt der Entnahmesonde, insbesondere der U-förmigen Entnahmesonde ausbilden. Die Entnahmesonde kann zusätzlich oder alternativ längliche Leitungsabschnitte aufweisen.

Die Entnahmeleitung kann eine Vielzahl an Leitungsabschnitten, insbesondere erste bis siebte Leitungsabschnitte aufweisen und die Entnahmeleitung kann derart ausgebildet und in der Gasleitung anordenbar sein, dass sich die Leitungsabschnitte zumindest teilweise in einem identischen und/oder unterschiedlichen Winkel zu der Strömungsrichtung erstrecken. Die Leitungsabschnitte können miteinander fluidisch verbunden sein.

Die Entnahmeleitung kann einen vorbestimmten Durchmesser aufweisen. Der vorbestimmte Durchmesser kann einem Durchmesser der kreisrund ausgebildeten Eingangs- und/oder Ausgangsöffnungen, insbesondere der Entnahme- und/oder Ausgabequerschnitte entsprechen, wobei die Eingangs- und/oder Ausgangsöffnungen den vorbestimmten Durchmesser aufweisen. Alternativ kann die Entnahmeleitung zwei, drei oder mehrere verschiedene Durchmesser aufweisen.

Die Entnahmeleitung kann derart ausgebildet und zumindest teilweise in der Gasleitung anordenbar sein, dass sich der erste Leitungsabschnitt von der Eingangsöffnung in einem ersten vorbestimmten Winkel zu der Strömungsrichtung erstreckt und sich ein Leitungsabschnitt, insbesondere der siebte Leitungsabschnitt von der Ausgangsöffnung in einem vierten vorbestimmten Winkel zu der Strömungsrichtung erstreckt, wobei die ersten und vierten Winkel unterschiedlich sind und insbesondere um 90° unterschiedlich zueinander sind. Die angegebenen Maße in Form des "°"-Zeichens der Winkel können Bogengrad oder Winkelmaß sein.

Die Entnahmeleitung kann derart ausgebildet und in der Gasleitung anordenbar sein, dass sich der zweite an den ersten Leitungsabschnitt angrenzende Leitungsabschnitt senkrecht zu der Strömungsrichtung erstreckt und sich der sechste an den siebten Leitungsabschnitt angrenzende Leitungsabschnitt senkrecht zu der Strömungsrichtung erstreckt.

Die Entnahmeleitung kann derart ausgebildet und in der Gasleitung anordenbar sein, dass sich der dritte an den zweiten Leitungsabschnitt angrenzende Leitungsabschnitt in einem zweiten vorbestimmten Winkel zu der Strömungsrichtung erstreckt und sich der fünfte an den sechsten Leitungsabschnitt angrenzende Leitungsabschnitt in einem dritten vorbestimmten Winkel zu der Strömungsrichtung erstreckt, wobei die zweiten und dritten Winkel unterschiedlich sind und insbesondere um 90° unterschiedlich zueinander sind.

Die Entnahmeleitung kann derart ausgebildet und in der Gasleitung anordenbar sein, dass der vierte an den dritten und fünften Leitungsabschnitt angrenzende Leitungsabschnitt der Entnahmeleitung U-förmig ausgebildet ist und sich insbesondere die beiden Längsstücke des U-förmigen Leitungsabschnitts senkrecht zu der Strömungsrichtung erstrecken. Die U-Form kann ein erstes, insbesondere geradlinig ausgebildetes Längsstück und ein zweites, insbesondere geradliniges Längsstück umfassen, die miteinander mittels eines gebogenen Stücks fluidisch verbunden sind.

Der erste Winkel kann zwischen 10° und 80°, insbesondere 45° aufweisen. Der zweite Winkel kann zwischen 100° und 170°, insbesondere 135° aufweisen. Der dritte Winkel kann zwischen 10° und 80°, insbesondere 45° aufweisen. Der vierte Winkel kann zwischen 100° und 170°, insbesondere 135° aufweisen. Die ersten und dritten Winkel können identisch sein. Die zweiten und vierten Winkel können identisch sein.

Mittels der vorgeschlagenen Entnahmesonde ist eine schnelle Ansprechzeit gewährleistet, da die Strömungsgeschwindigkeit des Gases in der Gasleitung genutzt wird. Zusätzlich ist eine Produktion der Entnahmesonde vereinfacht, da die meisten Komponenten dieser nicht aktiv sind bzw. nicht elektronisch und/oder mechanisch zu steuern sind. Zusätzlich wird die Gefahr einer Verstopfung bei einer entsprechenden Neigung des Leitungsabschnitts umfassend die Eingangsöffnung und einer Neigung des Leitungsabschnitts umfassend die Ausgangsöffnung verringert.

Die Schnittstelle kann an und/oder in einem bogenförmigen Abschnitt der U-förmigen Entnahmeleitung, des vierten Leitungsabschnitts und/oder einem länglichen Leitungsabschnitt angeordnet sein.

Die zumindest eine Messvorrichtung ist zur Vermessung des Gases ausgebildet. Dabei kann die zumindest eine Messvorrichtung zur Beschaffenheitsmessung des Gases ausgebildet sein. Weitere Messvorrichtungen des Systems können weitere Eigenschaften und/oder Parameter des Gases vermessen.

Das System kann zumindest eine Druckreduziervorrichtung, eine Leitung, die zum Leiten des Gases von der Schnittstelle zu der Druckreduziervorrichtung ausgebildet ist, wobei die Druckreduziervorrichtung dazu ausgebildet ist, einen Druck des zu der Druckreduziervorrichtung geleiteten Gases auf einen vorbestimmten Druck zu reduzieren, und eine Leitung, mittels der das Gas mit dem reduzierten Druck zu der Messvorrichtung leitbar ist, umfassen.

Die Gasleitung kann eine Erdgasleitung sein. Das Gas kann Erdgas sein und/oder umfassen.

Die Eingangsöffnung kann derart ausgebildet und in der Gasleitung anordenbar sein, dass das in der Gasleitung strömende Gas frontal auf die Eingangsöffnung trifft.

Die Eingangs- und/oder Ausgangsöffnungen können derart ausgebildet und in der Gasleitung anordenbar sein, dass diese auf einer im Wesentlichen gleichen Höhe, insbesondere derselben Höhe in der Gasleitung anordenbar sind. Die Höhe erstreckt sich entlang einer Höhenrichtung, welche senkrecht zu der Strömungsrichtung ist. Die Höhenrichtung kann parallel zu einer Richtung der Gravitationskraft sein. Mit anderen Worten können die Eingangs- und Ausgangsöffnungen eine im Wesentlichen gleiche Eintauchtiefe, insbesondere dieselbe Eintauchtiefe in die Gasleitung aufweisen, wobei sich die Eintauchtiefe ebenfalls entlang der Höhenrichtung erstreckt, jedoch im Vergleich zu der Höhe in die andere Richtung entlang der Höhenrichtung.

Die Entnahmesonde kann in Form einer Leitung und/oder eines Rohrs ausgebildet sein. Das mittels der Entnahmesonde entnommene Gas kann mittels zumindest eines Rohrs und/oder zumindest einer Leitung von der Schnittstelle zu der Druckreduziervorrichtung und mittels eines weiteren Rohrs und/oder einer weiteren Leitung von der Druckreduziervorrichtung zu der Messvorrichtung geleitet werden.

Die Entnahmesonde kann zwei, drei oder mehr Schnittstellen aufweisen, und/oder das System kann zwei, drei oder mehr Druckreduziervorrichtung und/oder Messvorrichtungen umfassent, wobei insbesondere mittels der zwei, drei oder mehr Schnittstellen das Gas zumindest teilweise von der Entnahmeleitung zu den zwei, drei oder mehr Druckreduziervorrichtungen und/oder Messvorrichtungen leitbar ist.

Die Eingangs- und/oder Ausgangsöffnung können derart in der Gasleitung anordenbar sein, dass diese entlang einer Anordnungsrichtung angeordnet sind, die sich parallel zu der Strömungsrichtung erstreckt.

Die Entnahmesonde kann vor und/oder nach der Schnittstelle der Entnahmeleitung, insbesondere stromabwärts entlang der Entnahmeleitung betrachtet nach der letzten Schnittstelle der zwei, drei oder mehr Schnittstellen und/oder vor der ersten Schnittstelle der zwei, drei oder mehr Schnittstellen zumindest ein Ventil, insbesondere ein Doppel-Block-and-Bleed-Ventil aufweisen. Das Doppel-Block-and-Bleed-Ventil kann zwei Absperrventile (Block) und ein Entlüftungsventil (Bleed) aufweisen. Falls das Absperrventil im Fehlerfall nicht mehr schließt, verhindert das zweite Absperrventil einen unkontrollierten Medienaustritt oder eine Kontamination des Verfahrens von außen.

Das System kann ein Gehäuse umfassen, das derart ausgebildet ist, dass die zumindest eine Druckreduziervorrichtung und/oder die zumindest eine Messvorrichtung in das Gehäuse aufgenommen sind, wobei das Gehäuse derart ausgebildet ist, dass die Entnahmesonde, insbesondere die Entnahmeleitung zumindest teilweise in das Gehäuse aufgenommen ist. Dabei kann insbesondere der bogenförmige Abschnitt und/oder der vierte Leitungsabschnitt zumindest teilweise in das Gehäuse aufgenommen sein.

Das System kann weiter zumindest eine Pumpeneinheit umfassen, die zum zumindest teilweisen Bereitstellen der Druckdifferenz und/oder Verstärken der Druckdifferenz ausgebildet ist. Die Pumpeneinheit kann derart ausgebildet sein, dass die Druckdifferenz derart ausbildbar ist, dass die vorbestimmte Strömungsgeschwindigkeit ausbildbar ist.

Die Entnahmesonde kann weiter ein Leitungsgehäuse umfassen, das zumindest abschnittsweise die Entnahmeleitung umfasst. Dabei kann das Leitungsgehäuse derart ausgebildet sein, dass das Leitungsgehäuse zumindest abschnittsweise in die Gasleitung, insbesondere eine entsprechende Öffnung der Gasleitung einführbar ist. Insbesondere kann das Leitungsgehäuse derart in der Gasleitung und/oder der Öffnung anordenbar sein, dass das Leitungsgehäuse fluidisch mit der Gasleitung und/oder der Öffnung abdichtet. Weiter kann das Leitungsgehäuse derart ausgebildet sein, dass dieses zusätzlich oder alternativ zumindest den U-förmigen Abschnitt der Entnahmesonde umfasst. Das Leitungsgehäuse kann zumindest abschnittsweise in das Gehäuse aufgenommen sein und/oder mit diesem verbunden sein.

Die Entnahmesonde ist derart ausgebildet, dass ein Staudruck das Gases an der Eingangsöffnung und ein dynamischer Druck an der Ausgangsöffnung ausbildbar ist, derart, dass die Druckdifferenz zwischen der Eingangs- und Ausgangsöffnung ausbildbar ist. Der erste Druck, insbesondere der Staudruck kann höher als der zweite Druck, insbesondere der dynamische Druck ausbildbar sein.

Die Aufgabe wird gemäß einem zweiten Aspekt durch eine Anordnung gelöst, umfassend ein System gemäß dem ersten Aspekt und die Gasleitung, wobei zumindest die Eingangsöffnung und die Ausgangsöffnung in der Gasleitung angeordnet sind.

Bevorzugte Ausführungsbeispiele werden exemplarisch anhand der beiliegenden Figuren erläutert. Es zeigen:
Fig. 1 ein System zur Beschaffenheitsmessung eines in einer Gasleitung strömenden Gases; und
Fig. 2 ein Ausführungsbeispiels einer Entnahmesonde für ein solches System.

In den Figuren sind gleiche oder im Wesentlichen funktionsgleiche beziehungsweise -ähnliche Elemente mit den gleichen Bezugszeichen bezeichnet.

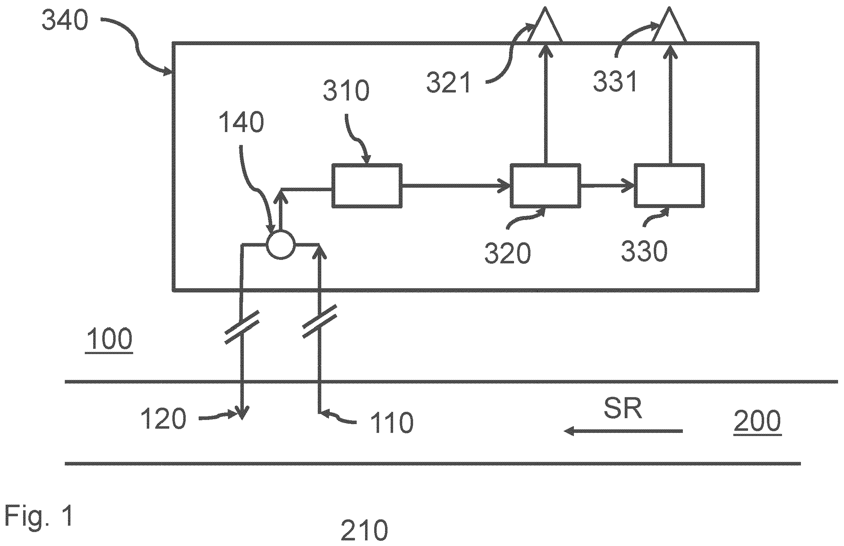

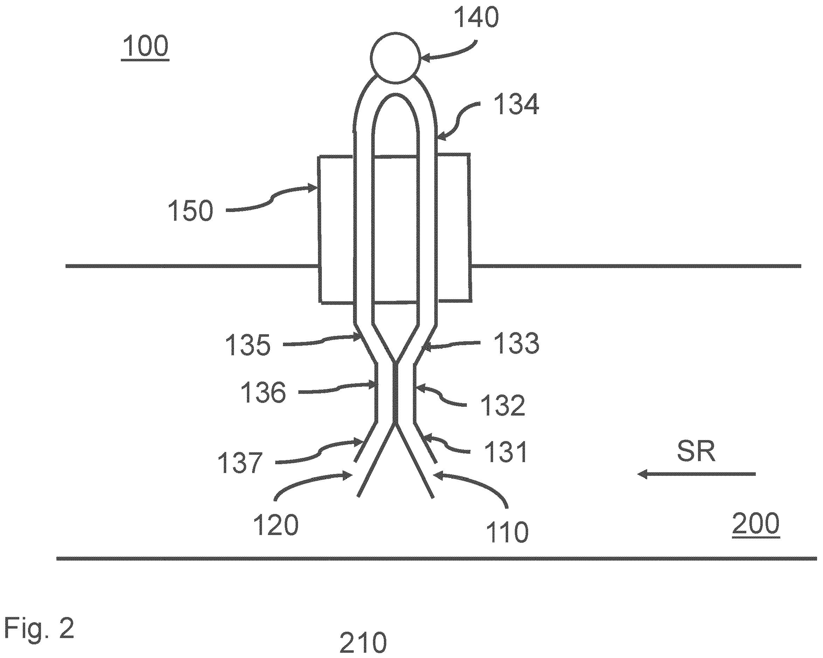

Fig. 1 zeigt ein System zur Beschaffenheitsmessung eines in einer Gasleitung 200 strömenden Gases, wobei das Gas entlang einer Strömungsrichtung SR mit einem vorbestimmten Druck und einer vorbestimmten Strömungsgeschwindigkeit strömt. Das System umfasst eine Entnahmesonde 100 zum Entnehmen von Gas aus der Gasleitung 200, wobei die Entnahmesonde 100 weiter zum zumindest teilweisen Zurückführen des Gases in die Gasleitung 200 ausgebildet ist. Die Pfeile in den Fig. 1 und 2 können Leitungen und/oder Rohre sein, die zum Leiten des Gases ausgebildet sind. Die Leitungen und/oder Rohre können die jeweiligen Einheiten des Systems fluidisch miteinander verbinden. Die Pfeile weisen dabei Pfeilrichtungen auf, die eine jeweilige Strömungsrichtung des Gases entlang der Leitungen und/oder Rohre angeben. Das System umfasst ein optionales Gehäuse 340, in das eine optionale Druckreduziervorrichtung 310, ein optionaler Bypass 320 und eine Messvorrichtung 330 aufgenommen sind. Weiter ist die Entnahmesonde 100 zumindest teilweise in das Gehäuse 340 aufgenommen.

Die Entnahmesonde 100 weist eine Schnittstelle 140 auf, welche mit der Druckreduziervorrichtung 310 fluidisch verbunden ist. Die Entnahmesonde 100 kann zwei, drei oder mehr Schnittstellen umfassen, wobei in der Fig. 1 nur eine gezeigt ist. Dabei ist ein Abschnitt der Entnahmesonde 100, an und/oder in dem die Schnittstelle 140 angeordnet ist, in das Gehäuse 340 aufgenommen. Mittels der Schnittstelle 140 kann ein vorbestimmter Anteil des mittels der Entnahmesonde 100 entnommenen Gases entnommen und an die Druckreduziervorrichtung 310 geleitet werden.

Aufgrund des hohen Drucks des Gases in der Gasleitung und damit auch in der Entnahmesonde 100 kann es für die Beschaffenheitsmessung des Gases notwendig sein, den Druck des Gases mittels der Druckreduziervorrichtung 310 zu verringern. Dies kann abhängig von der Art der Messvorrichtung und/oder der Vermessungsart sein. Anschließend kann das Gas mit reduziertem Druck zu dem optionalen Bypass 320 oder direkt zu der Messvorrichtung 330 geleitet werden. Der Bypass 320 ist dazu ausgebildet, einen Anteil des Gases mit niedrigem Druck weiter an die Messvorrichtung 330 und den restlichen Anteil an die Umgebung über den Ausgang 321 zu leiten. Der Bypass 320 kann dazu ausgebildet sein, einen Durchfluss des Gases durch den Bypass 320, die Messvorrichtung 330, einen Bypass-Auslass 321 und/oder einen Messvorrichtungsauslass 331 zu erhöhen, insbesondere bei einem Unterschreiten einer vorbestimmten Druckdifferenz zwischen einem Druck des Gases entlang einer jeweiligen Leitung stromaufwärts vor dem und/oder an dem Bypass 320 und einem Druck des Gases stromabwärts des Bypasses 320 bei und/oder an zumindest einem von der Messvorrichtung 330, dem Bypass-Auslass 321 und/oder dem Messvorrichtungsauslass 331.

Die Messvorrichtung 330 ist dazu ausgebildet, das entnommene Gas zu vermessen, insbesondere eine Beschaffenheit des Gases auszuführen. Dazu kann die Messvorrichtung 330 einen Gaschromatographen und einen Einlass für ein Trägergas aufweisen. Das Gehäuse 340 kann weiter eine das Trägergas bereitstellende Einheit umfassen. Das mittels der Messvorrichtung 330 vermessene Gas kann mittels des Ausgangs 331 an die Umgebung abgegeben werden.

Um eine Belastung der Umwelt möglichst gering zu halten, sollte die Menge des Gases, das an die Umgebung abgegeben wird, möglichst geringgehalten werden. Weiter wäre es vorteilhaft, möglichst wenig Gas aus der Gasleitung für die Beschaffenheitsmessung entnehmen zu müssen. Ebenfalls ist es wünschenswert, dass eine Distanz zwischen einer Entnahmestelle der Gasleitung 200 und des Systems flexibel gestaltet werden kann, insbesondere mehrere Meter aufweisen kann.

Diese und weitere Vorteile ergeben sich mit der Entnahmesonde 100, indem diese eine Eingangsöffnung 110 und eine Ausgangsöffnung 120 aufweist, die beide in der Gasleitung 200 angeordnet sind, wobei die Ausgangsöffnung 120 stromabwärts entlang der Strömungsrichtung SR des in der Gasleitung 200 strömenden Gases, insbesondere stromabwärts der Eingangsöffnung 110 angeordnet ist. Die Eingangsöffnung 110 ist stromaufwärts, insbesondere stromaufwärts der Ausgangsöffnung 120 entlang der Strömungsrichtung SR des in der Gasleitung 200 strömenden Gases angeordnet. Die Eingangs- und Ausgangsöffnungen 110, 120 bilden jeweils Endabschnitte der Entnahmesonde 100, welche miteinander mittels einer Entnahmeleitung der Entnahmesonde 100 fluidisch miteinander verbunden sind.

Dabei ist die Eingangsöffnung 110 durch einen Eingangsquerschnitt ausgebildet, der sich innerhalb einer Ebene erstreckt, die senkrecht zu der Strömungsrichtung SR angeordnet ist. Die Ausgangsöffnung 120 ist durch einen Ausgabequerschnitt ausgebildet, der parallel zu dieser Ebene ausgebildet ist. Dabei ist die Eingangsöffnung 110 dem in der Gasleitung 200 strömenden Gas zugewandt und die Ausgangsöffnung 120 dem strömenden Gas abgewandt. Durch diesen Aufbau der Entnahmesonde 100 strömt das Gas aus der Gasleitung über die Eingangsöffnung 110 in die Entnahmesonde. Dabei bildet sich ein Staudruck an der Eingangsöffnung 110, der höher als der Druck des Gases in der Gasleitung ist. Weiter bildet sich ein dynamischer Druck an der Ausgangsöffnung 120 aus, der niedriger als der Druck des Gases in der Gasleitung, insbesondere niedriger als der Staudruck ist. Folglich liegt eine Druckdifferenz zwischen Eingangs- und Ausgangsöffnung vor, was zu einer Strömung bzw. Strömungsgeschwindigkeit des Gases entlang der Entnahmesonde 100, insbesondere der Entnahmeleitung führt. Das Gas strömt mit dieser Strömungsgeschwindigkeit entlang der Entnahmeleitung und kann mittels der zumindest einen Schnittstelle 140 zumindest teilweise für Messzwecke entnommen werden. Der restliche Anteil des Gases strömt weiter zu der Ausgangsöffnung 120 und kann folglich zurück in die Gasleitung 200 geführt werden.

In der Fig. 1 ist durch die jeweiligen diagonal in Fig. 1 verlaufenden Doppelstriche stromabwärts nach der Eingangsöffnung 110 und stromaufwärts vor der Ausgangsöffnung 120 angezeigt, dass diese Leitungsabschnitte, insbesondere der Entnahmeleitung eine vorbestimmte Länge aufweisen können. Ebenfalls ist die Entnahmesonde 100, insbesondere die Entnahmeleitung nicht auf diese Form der Leitung eingeschränkt. Wie bereits eingangs erwähnt, wird auf besonders vorteilhafte Weise der Druck des Gases in der Gasleitung 200 genutzt, um mittels der Entnahmesonde 100 Gas aus der Gasleitung 200 entnehmen zu können, dieses mit einer vorbestimmten Strömungsgeschwindigkeit entlang der Entnahmesonde 100, insbesondere der Entnahmeleitung zu leiten und schließlich zumindest teilweise wieder über die Ausgangsöffnung 120 auszugeben. Je nach Druck des Gases in der Gasleitung 200 und/oder eines Aufbaus der Entnahmesonde 100, insbesondere der Entnahmeleitung kann das Gas in der Entnahmesonde 100 mit einer vorbestimmten Strömungsgeschwindigkeit strömen. Je nach Applikation des Systems und/oder einer Messaufgabe der Messvorrichtung 330 und/oder weiterer Aspekte, wie einer sicheren Entfernung zu der Gasleitung 200, kann ohne weitere Mittel mittels der Entnahmesonde 100 das Gas aus der Gasleitung 200 entnommen und zumindest teilweise zurückgeführt werden. Folglich kann eine Distanz zwischen Gasleitung 200 und Gehäuse 340 mehrere Meter betragen. Ein Aufbau der Entnahmesonde 100, insbesondere der Entnahmeleitung kann vorteilhaft durch eine Strömungssimulation bestimmt werden. Mögliche Parameter für diese Simulation können der Druck des Gases in der Gasleitung 200, ein Durchmesser der Entnahmeleitung und/oder der Eingangs- und Ausgangsöffnungen 110, 120 und eine voraussichtliche Distanz zu dem Gehäuse 340 und/oder der Schnittstelle 140 sein.

Sollte die Druckdifferenz zwischen dem Staudruck und dem dynamischen Druck und dem damit resultierenden Druck an der Eingangsöffnung und dem resultierenden Druck an der Ausgangsöffnung nicht ausreichend sein, kann das System weiter eine Pumpe umfassen, die dazu ausgebildet ist, die Druckdifferenz auf eine vorbestimmte Druckdifferenz zu erhöhen, derart, dass eine gewünschte Strömungsgeschwindigkeit des Gases entlang der Entnahmesonde 100 ausbildbar ist.

Weiter kann die Entnahmesonde 100 ein, zwei oder mehr Ventile, insbesondere Doppel-Block-and-Bleed-Ventil aufweisen, die entlang der Entnahmeleitung angeordnet sind. Dabei kann zumindest ein solches Ventil zwischen zwei Schnittstellen der Entnahmesonde 100 angeordnet sein.

Fig. 2 zeigt ein Ausführungsbeispiel einer Entnahmesonde 100. Die Entnahmesonde 100 weist eine Eingangsöffnung 110 zum Entnehmen von Gas aus der Gasleitung 200 auf. Gemäß der Fig. 2 ist die Entnahmeöffnung 110 derart ausgebildet und in der Gasleitung 200 anordenbar, dass das Gas frontal auf die Entnahmeöffnung 110 trifft. Entsprechend kann ein Entnahmequerschnitt der Eingangsöffnung 110 senkrecht zu der Strömungsrichtung SR ausgebildet sein. Dies kann für eine maximale Entnahme an Gas mittels der Entnahmeöffnung 110 sorgen.

Die Entnahmesonde 100 weist weiter eine Ausgangsöffnung 120 auf, welche stromabwärts entlang der Strömungsrichtung SR nach der Eingangsöffnung 110 in der Gasleitung 200 anordenbar ist. Mittels der Ausgangsöffnung 120 kann zumindest teilweise das mittels der Eingangsöffnung 110 entnommene Gas zurück in die Gasleitung 200 geführt werden. Dabei können die Eingangs- und Ausgangsöffnung derart in der Gasleitung 200 anordenbar sein, dass diese entlang einer Anordnungsrichtung angeordnet sind, die sich parallel zu der Strömungsrichtung SR erstreckt. Teilweise bedeutet in diesem Zusammenhang, dass ein Anteil des mittels der Eingangsöffnung 110 entnommenen Gases über die Schnittstelle 140 an die Druckreduziervorrichtung 310 geleitet wird.

Dazu sind die Eingangsöffnung 110 und die Ausgangsöffnung 120 fluidisch mittels einer Entnahmeleitung verbunden, welche Leitungsabschnitte 131 bis 137 aufweist. Die Entnahmesonde 100 kann insbesondere als "Loop"- oder Schleifen-Entnahmesonde 100 bezeichnet werden, da, wie in Fig. 2 gezeigt, das Gas mittels der Eingangsöffnung 110 entnommen, über die Leitungsabschnitte 131-137 entlang einer Loop bzw. einer Schleife zu der Ausgangsöffnung 120 geführt wird. Mittels der Schnittstelle 140 kann an einem vierten Leitungsabschnitt 134 ein Teil des Gases entnommen und weiter an die Druckreduziervorrichtung 310 geleitet werden. Das restliche Gas kann weiter zu der Ausgangsöffnung 120 geführt und in die Gasleitung 200 zurückgeführt werden.

Vorteilhafterweise wird der Druck und die Strömungsgeschwindigkeit des Gases in der Gasleitung 200 wie zuvor beschrieben genutzt, damit das Gas durch die Eingangsöffnung 110 entlang der Entnahmeleitung zu der Ausgangsöffnung 120 strömt.

Die Entnahmeleitung weist die Leitungsabschnitte 131 bis 137 auf. Der erste Leitungsabschnitt 131 ist ein erster Endabschnitt der Entnahmeleitung und weist die Eingangsöffnung 110 auf. Dabei ist der erste Leitungsabschnitt 131 derart ausgebildet und in der Gasleitung 200 anordenbar, dass dieser einen vorbestimmten Winkel zwischen 10° und 80°, insbesondere 45° zu der Strömungsrichtung aufweist.

Der Winkel zwischen einem Leitungsabschnitt und der Strömungsrichtung SR kann ein Winkel zwischen einer Außenfläche, einer Innenfläche und/oder einer Haupterstreckungsrichtung des Leitungsabschnitts und der Strömungsrichtung SR sein. Gemäß der Fig. 2 weist die Entnahmeleitung, insbesondere die Leitungsabschnitte 131-137 einen vorbestimmten Durchmesser auf.

Weiter können einer oder mehrere der Leitungsabschnitte 131-137 einen runden, insbesondere kreisrunden Querschnitt aufweisen. Die Leitungsabschnitte 131-133 und 135-137 können eine zylindrische Form aufweisen. Die zylindrische Form kann sich entlang der Haupterstreckungsrichtung mit einer vorbestimmten Zylinderhöhe und senkrecht zu der Haupterstreckungsrichtung mit einem vorbestimmten Zylinderradius erstrecken. Der Leitungsabschnitt 134 ist U-förmig, gemäß der Fig. 2 in Form eines umgedrehten U dargestellt. Dabei weist der Leitungsabschnitt 134 zwei längliche, sich senkrecht zu der Strömungsrichtung SR erstreckende Längsstücke auf, welche mit den Leitungsabschnitten 133, 135 fluidisch verbunden sind. Diese Längsstücke können zylinderförmig ausgebildet sein. Weiter weist der Leitungsabschnitt 134 zwischen den beiden Längsstücken ein bogenförmiges Stück auf, wobei die Schnittstelle 140 an und/oder in dem bogenförmigen Stück angeordnet ist. Die Schnittstelle 140 kann dazu ausgebildet sein, fluidisch mit der Entnahmeleitung abzudichten und/oder zumindest teilweise Gas weiter an die Druckreduziervorrichtung 310 zu leiten.

Gemäß der Fig. 2 ist mit dem ersten Leitungsabschnitt 131 ein zweiter Leitungsabschnitt 132 fludisch verbunden, der sich senkrecht zu der Strömungsrichtung SR erstreckt. Mit dem zweiten Leitungsabschnitt 132 ist ein dritter Leitungsabschnitt 133 fluidisch verbunden, der in einem vorbestimmten Winkel, insbesondere zwischen 100° und 170° zu der Strömungsrichtung SR angeordnet ist. Entlang der Entnahmeleitung betrachtet sind die Leitungsabschnitte 135-137 spiegelverkehrt zu den Leitungsabschnitten 131-133 angeordnet, wobei die Spiegelung entlang einer Achse erfolgt, die sich senkrecht zu der Strömungsrichtung SR erstreckt. Die Leitungsabschnitte 131-137 sind in Reihe angeordnet und miteinander fluidisch verbunden. Mittels der in Fig. 2 gezeigten Anordnung der Leitungsabschnitte 131-137 ist ein Druckverlust entlang der Entnahmesonde 100 besonders gering.

Weiter zeigt die Fig. 2 ein Leitungsgehäuse 150, das zumindest abschnittsweise die Entnahmeleitung umfasst. Dabei ist das Leitungsgehäuse 150 derart ausgebildet, dass das Leitungsgehäuse 150 zumindest abschnittsweise in die Gasleitung 200 einführbar ist und/oder mit diesem fluidisch abdichtet. Insbesondere kann das Leitungsgehäuse 150 derart in der Gasleitung 200 anordenbar sein, dass das Leitungsgehäuse 150 fluidisch mit einer entsprechenden Öffnung der Gasleitung 200 abdichtet. Weiter kann das Leitungsgehäuse 150 derart ausgebildet sein, dass dieses zusätzlich oder alternativ zumindest den U-förmigen Abschnitt der Entnahmesonde 100 umfasst.

### BEZUGSZEICHEN

- 100: Entnahmesonde
- 110: Eingangsöffnung
- 120: Ausgangsöffnung
- 131-137: Leitungsabschnitte der Entnahmeleitung
- 140: Schnittstelle
- 150: Leitungsgehäuse
- 200: Gasleitung
- SR: Strömungsrichtung des Gases in der Gasleitung
- 310: Druckreduziervorrichtung
- 320: Bypass-Vorrichtung
- 321: Bypass-Auslass
- 330: Messvorrichtung
- 331: Messvorrichtungsauslass

## Patentansprüche

1. System zur Vermessung eines in einer Gasleitung (200) strömenden Gases, umfassend:
eine Entnahmesonde (100) mit einer Eingangsöffnung (110) zum Entnehmen eines in einer Gasleitung (200) strömenden Gases, einer Ausgangsöffnung (120) zum zumindest teilweisen Ausgeben des entnommenen Gases in die Gasleitung (200) und einer Entnahmeleitung, die zumindest die Eingangsöffnung (110) und die Ausgangsöffnung (120) fluidisch verbindet,
wobei die Entnahmesonde (100) derart ausgebildet ist, dass zumindest die Eingangsöffnung (110) und die Ausgangsöffnung (120) in der Gasleitung (200) anordenbar sind,
wobei die Entnahmesonde (100) derart ausgebildet ist, dass die Ausgangsöffnung (120) entlang einer Strömungsrichtung (SR) des in der Gasleitung (200) strömenden Gases stromabwärts der Eingangsöffnung (110) anordenbar ist;
zumindest eine Messvorrichtung (330) zum Vermessen des Gases,
wobei die Entnahmeleitung zumindest eine Schnittstelle (140) aufweist, über die zumindest teilweise das Gas zu der Messvorrichtung (330) leitbar ist,
**dadurch gekennzeichnet, dass**
die Entnahmesonde (100) derart ausgebildet ist, dass ein Staudruck des Gases an der Eingangsöffnung (110) und ein dynamischer Druck an der Ausgangsöffnung (120) ausbildbar ist, derart, dass eine Druckdifferenz zwischen der Eingangs- und Ausgangsöffnung (110, 120) ausbildbar ist,
wobei die Druckdifferenz derart ausbildbar ist, dass das mittels der Entnahmesonde (100) entnommene Gas mit einer vorbestimmten Strömungsgeschwindigkeit durch die Entnahmesonde (100) strömt.

2. System nach Anspruch 1,
wobei die Eingangsöffnung (110) derart ausgebildet und in der Gasleitung (200) anordenbar ist, dass das in der Gasleitung (200) strömende Gas aufgrund einer Strömungsgeschwindigkeit des Gases in Richtung der Strömungsrichtung (SR) mittels der Eingangsöffnung (110) aus der Gasleitung (200) entnehmbar ist.

3. System nach einem der vorherigen Ansprüche,
wobei die vorbestimmte Strömungsgeschwindigkeit basierend auf einer Strömungsstrecke und/oder einer Messaufgabe der Messvorrichtung bestimmt ist,
wobei die Strömungsstrecke eine Kreislaufstrecke und/oder eine Messstrecke umfasst,
wobei die Kreislaufstrecke eine Strecke ist, die das mittels der Eingangsöffnung (110) entnommene Gas von der Eingangsöffnung (110) entlang der Entnahmeleitung zu der Ausgangsöffnung (120) strömt,
wobei die Messstrecke eine Strecke ist, die das mittels der Eingangsöffnung (110) entnommene Gas von der Eingangsöffnung (110) zu der der Messvorrichtung (330) strömt.

4. System nach einem der vorherigen Ansprüche,
wobei die Eingangsöffnung (110) durch einen Entnahmequerschnitt zum Entnehmen des Gases aus der Gasleitung (200) und/oder die Ausgangsöffnung (120) durch einen Ausgabequerschnitt zum zumindest teilweisen Ausgeben des entnommenen Gases in die Gasleitung (200) ausgebildet sind,
wobei die Entnahmesonde (100) derart ausgebildet ist, dass der Entnahmequerschnitt und/oder der Ausgabequerschnitt senkrecht zu der Strömungsrichtung (SR) in der Gasleitung (200) anordenbar sind.

5. System nach einem der vorherigen Ansprüche,
wobei die Entnahmesonde (100) derart ausgebildet ist, dass der Entnahmequerschnitt dem entlang der Strömungsrichtung (SR) in der Gasleitung (200) strömenden Gas zugewandt und/oder der Ausgabequerschnitt dem entlang der Strömungsrichtung (SR) in der Gasleitung (200) strömenden Gas abgewandt ist.

6. System nach einem der vorherigen Ansprüche,
wobei die Entnahmesonde (100) vor und/oder nach der Schnittstelle (140) der Entnahmeleitung (100) ein Ventil, insbesondere ein Doppel-Block-and-Bleed-Ventil aufweist.

7. System nach einem der vorherigen Ansprüche, weiter umfassend:
zumindest eine Druckreduziervorrichtung (310);
eine Leitung, die zum Leiten des Gases von der Schnittstelle (140) zu der Druckreduziervorrichtung (310) ausgebildet ist,
wobei die Druckreduziervorrichtung (310) dazu ausgebildet ist, einen Druck des zu der Druckreduziervorrichtung (310) geleiteten Gases auf einen vorbestimmten Druck zu reduzieren;
eine Leitung, mittels der das Gas mit dem reduzierten Druck zu der Messvorrichtung (330) leitbar ist.

8. System nach Anspruch 7, weiter umfassend:
ein Gehäuse (340), das derart ausgebildet ist, dass die Druckreduziervorrichtung (310) in das Gehäuse (340) aufgenommen ist,
wobei das Gehäuse (340) derart ausgebildet ist, dass die Entnahmesonde (100), insbesondere die Entnahmeleitung zumindest teilweise in das Gehäuse (340) aufgenommen ist.

9. System nach einem der vorherigen Ansprüche, weiter umfassend:
das Gehäuse (340), das derart ausgebildet ist, dass die Messvorrichtung (330) in das Gehäuse (340) aufgenommen ist,
wobei das Gehäuse (340) derart ausgebildet ist, dass die Entnahmesonde (100), insbesondere die Entnahmeleitung zumindest teilweise in das Gehäuse (340) aufgenommen ist.

10. System nach einem der vorherigen Ansprüche, weiter umfassend:
zumindest eine Pumpeneinheit, die zum zumindest teilweisen Bereitstellen der Druckdifferenz ausgebildet ist.

11. System nach einem der vorherigen Ansprüche,
wobei die Pumpeneinheit entlang der Entnahmeleitung stromabwärts der Schnittstelle (140) angeordnet ist.

12. System nach einem der vorherigen Ansprüche in Kombination mit Anspruch 7,
wobei die Entnahmeleitung zwei, drei oder mehr Schnittstellen (140) aufweist, und/oder
wobei das System zwei, drei oder mehr Druckreduziervorrichtungen (310) und/oder Messvorrichtungen (330) umfasst,
wobei insbesondere mittels der zwei, drei oder mehr Schnittstellen (140) das Gas zumindest teilweise von der Entnahmeleitung zu den zwei, drei oder mehr Druckreduziervorrichtungen (310) und/oder Messvorrichtungen (330) leitbar ist.

13. Anordnung umfassend ein System nach einem der vorherigen Ansprüche und eine Gasleitung (200), wobei zumindest die Eingangsöffnung (110) und die Ausgangsöffnung (120) in der Gasleitung (200) angeordnet sind.

## Claims

1. System for measuring a gas flowing in a gas line (200), comprising:
an extraction probe (100) having an inlet opening (110) for extracting a gas flowing in a gas line (200), an outlet opening (120) for at least partially outputting the extracted gas into the gas line (200), and an extraction line fluidically connecting at least the inlet opening (110) and the outlet opening (120),
wherein the extraction probe (100) is configured such that at least the inlet opening (110) and the outlet opening (120) are arrangeable in the gas line (200),
wherein the extraction probe (100) is configured such that the outlet opening (120) is arrangeable downstream of the inlet opening (110) along a flow direction (SR) of the gas flowing in the gas line (200);
at least one measuring device (330) for measuring the gas,
wherein the extraction line has at least one interface (140) via which the gas is at least partially directable to the measuring device (330),
**characterized in that** the extraction probe (100) is configured such that a stagnation pressure of the gas is generatable at the inlet opening (110) and a dynamic pressure is generatable at the outlet opening (120), such that a pressure difference between the inlet and outlet openings (110, 120) is generatable,
wherein the pressure difference is generatable such that the gas extracted by means of the extraction probe (100) flows through the extraction probe (100) at a predetermined flow velocity.

2. System according to claim 1,
wherein the inlet opening (110) is configured and arrangeable in the gas line (200) such that the gas flowing in the gas line (200) is extractable from the gas line (200) by means of the inlet opening (110) due to a flow velocity of the gas in the direction of the flow direction (SR).

3. System according to any of the previous claims,
wherein the predetermined flow velocity is determined based on a flow path and/or a measuring objective of the measuring device,
wherein the flow path comprises a circulation path and/or a measurement path,
wherein the circulation path is a path along which the gas extracted by means of the inlet opening (110) flows from the inlet opening (110) along the extraction line to the outlet opening (120),
wherein the measurement path is a path along which the gas extracted by means of the inlet opening (110) flows from the inlet opening (110) to the measuring device (330).

4. System according to any of the previous claims,
wherein the inlet opening (110) is configured by an extraction cross-section for extracting the gas from the gas line (200) and/or the outlet opening (120) is configured by an output cross-section for at least partially outputting the extracted gas into the gas line (200),
wherein the extraction probe (100) is configured such that the extraction cross-section and/or the output cross-section are arrangeable perpendicular to the flow direction (SR) in the gas line (200).

5. System according to any of the previous claims,
wherein the extraction probe (100) is configured such that the extraction cross-section faces the gas flowing along the flow direction (SR) in the gas line (200) and/or the output cross-section faces away from the gas flowing along the flow direction (SR) in the gas line (200).

6. System according to any of the previous claims,
wherein the extraction probe (100) comprises a valve, in particular a double block-and-bleed valve, upstream and/or downstream of the interface (140) of the extraction line (100).

7. System according to any of the previous claims, further comprising:
at least one pressure reducing device (310);
a line configured to direct the gas from the interface (140) to the pressure reducing device (310),
wherein the pressure reducing device (310) is configured to reduce a pressure of the gas directed to the pressure reducing device (310) to a predetermined pressure;
a line by means of which the gas at the reduced pressure is directable to the measuring device (330).

8. System according to claim 7, further comprising:
a housing (340) configured such that the pressure reducing device (310) is incorporated into the housing (340),
wherein the housing (340) is configured such that the extraction probe (100), in particular the extraction line, is at least partially incorporated into the housing (340).

9. System according to any of the previous claims, further comprising:
the housing (340), configured such that the measuring device (330) is incorporated into the housing (340),
wherein the housing (340) is configured such that the extraction probe (100), in particular the extraction line, is at least partially incorporated into the housing (340).

10. System according to any of the previous claims, further comprising:
at least one pump unit configured to at least partially provide the pressure difference.

11. System according to any of the previous claims,
wherein the pump unit is arranged along the extraction line downstream of the interface (140).

12. System according to any of the previous claims in combination with claim 7,
wherein the extraction line comprises two, three or more interfaces (140), and/or
wherein the system comprises two, three or more pressure reducing devices (310) and/or measuring devices (330),
wherein in particular by means of the two, three or more interfaces (140) the gas is at least partially directable from the extraction line to the two, three or more pressure reducing devices (310) and/or measuring devices (330).

13. Arrangement comprising a system according to any of the previous claims and a gas line (200), wherein at least the inlet opening (110) and the outlet opening (120) are arranged in the gas line (200).

## Revendications

1. Système de mesure d'un gaz circulant dans une conduite de gaz (200), comprenant :
une sonde de prélèvement (100) comportant un orifice d'entrée (110) destiné à prélever un gaz circulant dans une conduite de gaz (200), un orifice de sortie (120) destiné à délivrer au moins partiellement le gaz prélevé dans la conduite de gaz (200) et une conduite de prélèvement qui relie fluidiquement au moins l'orifice d'entrée (110) et l'orifice de sortie (120),
la sonde de prélèvement (100) étant conçue de telle sorte qu'au moins l'orifice d'entrée (110) et l'orifice de sortie (120) puissent être disposés dans la conduite de gaz (200),
la sonde de prélèvement (100) étant conçue de telle sorte que l'orifice de sortie (120) puisse être disposé en aval de l'orifice d'entrée (110) dans un sens d'écoulement (SR) du gaz circulant dans la conduite de gaz (200) ;
au moins un dispositif de mesure (330) destiné à mesurer le gaz,
la conduite de prélèvement comportant au moins une interface (140) par laquelle le gaz peut être acheminé, au moins en partie, vers le dispositif de mesure (330),
**caractérisé en ce que** la sonde de prélèvement (100) est conçue de telle sorte qu'une pression d'arrêt du gaz puisse être générée au niveau de l'orifice d'entrée (110) et une pression dynamique de vitesse au niveau de l'orifice de sortie (120) puissent se former, de telle sorte qu'une différence de pression entre les orifices d'entrée et de sortie (110, 120) puisse se former,
la différence de pression pouvant être formée de telle sorte que le gaz prélevé au moyen de la sonde de prélèvement (100) circule à travers la sonde de prélèvement (100) à une vitesse d'écoulement prédéterminée.

2. Système selon la revendication 1,
l'orifice d'entrée (110) étant conçu et pouvant être disposé dans la conduite de gaz (200) de telle sorte que le gaz circulant dans la conduite de gaz (200) puisse être prélevé de la conduite de gaz (200) au moyen de l'orifice d'entrée (110) en raison d'une vitesse d'écoulement du gaz dans la direction du sens d'écoulement (SR).

3. Système selon l'une des revendications précédentes,
la vitesse d'écoulement prédéterminée étant déterminée sur la base d'un trajet d'écoulement et/ou d'une tâche de mesure du dispositif de mesure,
le trajet d'écoulement comprenant un trajet circulaire et/ou un trajet de mesure, le trajet circulaire étant un trajet par lequel le gaz prélevé par l'orifice d'entrée (110) circule depuis l'orifice d'entrée (110) vers l'orifice de sortie (120) le long de la conduite de prélèvement,
le trajet de mesure étant un trajet que parcourt le gaz prélevé au moyen de l'orifice d'entrée (110) depuis l'orifice d'entrée (110) jusqu'au dispositif de mesure (330).

4. Système selon l'une des revendications précédentes,
l'orifice d'entrée (110) étant formé par une section de prélèvement destinée à prélever le gaz depuis la conduite de gaz (200) et/ou l'orifice de sortie (120) étant formé par une section de délivrance destinée à délivrer au moins partiellement le gaz prélevé, dans la conduite de gaz (200),
la sonde de prélèvement (100) étant conçue de telle sorte que la section de prélèvement et/ou la section de délivrance puissent être disposées perpendiculairement au sens d'écoulement (SR) dans la conduite de gaz (200).

5. Système selon l'une des revendications précédentes,
la sonde de prélèvement (100) étant conçue de telle sorte que la section de prélèvement soit orientée vers le gaz circulant dans la conduite de gaz (200) dans le sens d'écoulement (SR) et/ou que la section de délivrance soit orientée à l'opposé u gaz circulant dans la conduite de gaz (200) dans le sens d'écoulement (SR).

6. Système selon l'une des revendications précédentes,
la sonde de prélèvement (100) comportant, en amont et/ou en aval de l'interface (140) de la conduite de prélèvement (100), une vanne, en particulier une vanne double d'isolement avec purge.

7. Système selon l'une des revendications précédentes, comportant en outre :
au moins un dispositif de réduction de pression (310) ;
une conduite qui est conçue pour acheminer le gaz depuis l'interface (140) vers le dispositif de réduction de pression (310),
le dispositif de réduction de pression (310) étant conçu pour réduire à une pression prédéterminée une pression du gaz acheminé vers le dispositif de réduction de pression (310) ;
une conduite au moyen de laquelle le gaz à la pression réduite peut être acheminé vers le dispositif de mesure (330).

8. Système selon la revendication 7, comportant en outre :
un boîtier (340) qui est conçu de telle sorte que le dispositif de réduction de pression (310) soit logé dans le boîtier (340),
le boîtier (340) étant conçu de telle sorte que la sonde de prélèvement (100), en particulier la conduite de prélèvement, soit au moins partiellement logée dans le boîtier (340).

9. Système selon l'une des revendications précédentes, comportant en outre :
le boîtier (340), qui est conçu de telle sorte que le dispositif de mesure (330) soit logé dans le boîtier (340),
le boîtier (340) étant conçu de telle sorte que la sonde de prélèvement (100), en particulier la conduite de prélèvement, soit au moins partiellement logée dans le boîtier (340).

10. Système selon l'une des revendications précédentes, comportant en outre :
au moins une unité de pompe qui est conçue pour fournir, au moins en partie, la différence de pression.

11. Système selon l'une des revendications précédentes,
l'unité de pompe étant disposée en aval de l'interface (140) le long de la conduite de prélèvement.

12. Système selon l'une des revendications précédentes en combinaison avec la revendication 7,
la conduite de prélèvement comportant deux, trois ou davantage d'interfaces (140), et/ou
le système comprenant deux, trois ou davantage de dispositifs de réduction de pression (310) et/ou dispositifs de mesure (330),
le gaz pouvant être acheminé, au moins en partie, au moyen des deux, trois ou davantage d'interfaces (140) de la conduite de prélèvement vers les deux, trois ou davantage de dispositifs de réduction de pression (310) et/ou dispositifs de mesure (330).

13. Ensemble comprenant un système selon l'une des revendications précédentes et une conduite de gaz (200), au moins l'orifice d'entrée (110) et l'orifice de sortie (120) étant disposés dans la conduite de gaz (200).
